# EUROPÄISCHE PATENTSCHRIFT

(11) **EP 0 794 946 B1**
(45) Veröffentlichungstag und Bekanntmachung des Hinweises auf die Patenterteilung: **20.12.2006**
(21) Anmeldenummer: 95937841.5
(22) Anmeldetag: 25.10.1995
(51) Int. Cl.: C07D 239/46, C07D 239/42, A01N 47/36, C07D 251/46, C07D 251/16, C07D 251/42, C07D 405/12, C07D 409/12

(54) **ALKYLIDENHYDRAZINOPHENYLSULFONYLHARNSTOFFE, VERFAHREN ZU IHRER HERSTELLUNG UND IHRE VERWENDUNG ALS HERBIZIDE UND PFLANZENWACHSTUMSREGULATOREN**
ALKYLIDENEHYDRAZINOPHENYLSULPHONYL UREAS, METHODS OF PREPARING THEM AND THEIR USE AS HERBICIDES AND PLANT-GROWTH REGULATORS
ALKYLIDENE-HYDRAZINOPHENYLSULFONYL UREES, LEUR PROCEDE DE FABRICATION ET LEUR UTILISATION COMME HERBICIDES ET REGULATEURS DE CROISSANCE VEGETALE

(30) Priorität: 28.11.1994 DE 4442229
(43) Veröffentlichungstag der Anmeldung: 17.09.1997
(73) Patentinhaber: Bayer CropScience GmbH, 65929 Frankfurt/Main (DE)
(72) Erfinder: KEHNE, Heinz, D-65719 Hofheim (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: PCT/EP1995/004181
(87) Internationale Veröffentlichungsnummer: WO 1996/016944

(56) Entgegenhaltungen:
- EP-A- 0 382 437
- EP-A- 0 562 575

## Beschreibung

Es ist bekannt, daß Phenylsulfonylharnstoffe mit Hydrazin-Partialstrukturen herbizide Eigenschaften besitzen. Dabei handelt es sich vornehmlich um Hydrazone (EP-A-382 437, EP-A-562 575) oder um Heterocyclen mit inkorporierter Hydrazinstruktur (EP-A-382 436, EP-A-384 602).

Überraschenderweise wurden nun Phenylsulfonylharnstoffe mit bestimmten substituierten Hydrazonresten gefunden, die sich besonders gut als Herbizide oder Pflanzenwachstumsregulatoren eignen.

Gegenstand der vorliegenden Erfindung sind Verbindungen der Formel (I) oder deren Salze, 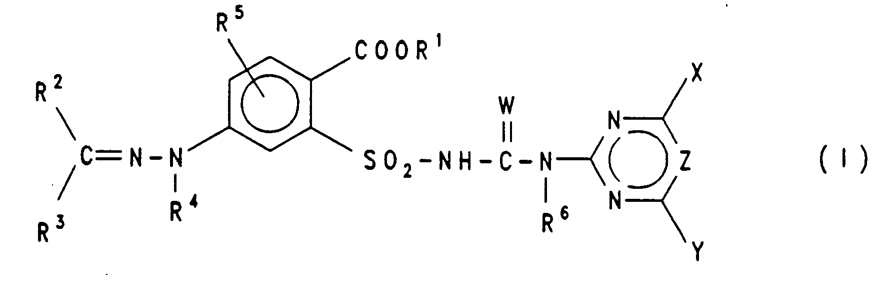 worin
- R¹: H oder einen Kohlenwasserstoffrest, der unsubstituiert oder substituiert ist, oder einen Heterocyclylrest, der unsubstituiert oder substituiert ist, wobei jeder der letztgenannten beiden Reste inklusive Substituenten vorzugsweise 1 bis 20 C-Atome aufweist,
- R²: Phenyl unsubstituiert oder substituiert und
- R³: (C₃-C₆)Cycloalkyl, unsubstituiert oder substituiert, oder
- R²: Wasserstoff oder (C₁-C₄)Alkyl und
- R³: Phenyl, das durch einen oder mehrere Reste aus der Gruppe (C₁-C₄)Haloalkyl, substituiert ist, oder
- R²: (C₁-C₄)Alkyl und
- R³: CF₃ oder
- R², R³: gemeinsam mit dem Methylen-C-atom einen Cycloalkylen- oder Heterocyclylenrest mit 4 bis 8 Ringatomen, der unsubstituiert oder substituiert ist, sowie
- R⁴: H, einen unsubstituierten oder substituierten, aliphatischen Kohlenwasserstoffrest mit 1 bis 6 C-Atomen im Kohlenwasserstoffteil und ein oder mehreren Substituenten, wobei die Substituenten aus der Gruppe Halogen, (C₁-C₄)Alkoxy, (C₁-C₄)Alkylthio, (C₁-C₄)Alkylsulfonyl, [(C₁-C₄)Alkyl]carbonyl, [(C₁-C₄)-Alkoxy]carbonyl, CN, substituiertes und unsubstituiertes Phenyl und (C₃-C₆)Cycloalkyl ausgewählt sind, oder (C₂-C₆)Alkenyl oder (C₂-C₆)Alkinyl oder einen Acylrest mit vorzugsweise 1 bis 20 C-Atomen,
- R⁵: H, Halogen, NO₂, CN, (C₁-C₄)Alkyl, (C₁-C₄)Alkoxy, [(C₁-C₄)Alkyl]-carbonyl oder [(C₁-C₄)Alkoxy]carbonyl, wobei jeder der letztgenannten vier Reste unsubstituiert oder im Alkylteil durch ein oder mehrere Halogenatome substituiert ist,
- R⁶: H oder (C₁-C₄)Alkyl, vorzugsweise H oder CH₃,
- W: ein Sauerstoff- oder Schwefelatom, vorzugsweise ein Sauerstoffatom,
- X, Y: unabhängig voneinander H, Halogen, (C₁-C₄)Alkyl, (C₁-C₄)Alkoxy, (C₁-C₄)Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Mono- oder Di-[(C₁-C₄)alkyl]-amino, (C₃-C₆)Cycloalkyl, (C₂-C₅)Alkenyl, (C₂-C₅)Alkinyl, (C₂-C₅)-Alkenyloxy oder (C₂-C₅)Alkinyloxy und
- Z: CH oder N
bedeuten.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen. Ebenso kann Salzbildung durch Anlagerung einer Säure an basischen Gruppen, wie z.B. Amino und Alkylamino, erfolgen. Geeignete Säuren hierfür sind starke anorganische und organische Säuren, beispielsweise HCl, HBr, H₂SO₄ oder HNO₃.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethytbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methyfprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Alkenyl in der Form "(C₃-C₄)Alkenyl" oder "(C₃-C₆)Alkenyl" bedeutet vorzugsweise einen Alkenylrest mit 3 bis 4 bzw. 3 bis 6 C-Atomen, bei dem die Doppelbindung nicht zwischen C-1 und C-2 liegt (C-1 ist die Position mit "yl").

Cycloalkyl bedeutet ein carbocyclisches, gesättigtes Ringsystem mit 3-8 C-Atomen, z.B. Cyclopropyl, Cyclopentyl oder Cyclohexyl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl; vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl; entsprechendes gilt für einen Kohlenwasserstoffrest in einem Kohlenwasserstoffoxyrest.

Ein heterocyclischer Rest oder Ring (Heterocyclyl) kann gesättigt, ungesättigt oder heteroaromatisch sein; er enthält vorzugsweise ein oder mehrere Heteroeinheiten im Ring, vorzugsweise aus der Gruppe N, 0, S, SO, SO₂; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroeinheiten. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.
Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono-und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, mund p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein Acylrest bedeutet den Rest einer organischen Säure, z.B. den Rest einer Carbonsäure und Reste davon abgeleiteter Säuren wie der Thiocarbonsäure, gegebenenfalls N-substituierten Iminocarbonsäuren oder den Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie (C₁-C₄-Alkyl)-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die vorstehenden Beispiele für Reste oder Restebereiche, die unter die allgemeinen Begriffe wie "Alkyl", "Acyl", "substituierten Reste" etc., fallen bedeuten keine vollständige Aufzählung. Die allgemeinen Begriffe umfassen auch die weiter unten angeführten Definitionen für Restebereiche in Gruppen bevorzugter Verbindungen, insbesondere Restebereiche, weiche spezifische Reste aus den Tabellenbeispielen umfassen.

Vor allem aus den Gründen der höheren herbiziden Wirkung, besseren Selektivität und/oder besseren Herstellbarkeit sind Verbindungen der Formel (I) oder deren Salze von besonderem Interesse, worin
- R¹: H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio und [(C₁-C₄)Alkoxy]carbonyl substituiert ist, oder (C₃-C₆)Cycloalkyl, (C₃-C₆)Cycloalkyl(C₁-C₃)alkyl, Heterocyclyl mit 3 bis 6 Ringatomen oder Heterocyclyl-(C₁-C₃)alkyl mit 3 bis 6 Ringatomen, wobei jeder der letztgenannten 4 Reste unsubstituiert oder durch einen oder mehrere Reste aus der Gruppe Halogen, (C₁-C₄)Alkyl und (C₁-C₄)Alkoxy substituiert ist,
- R²: Phenyl unsubstituiert oder durch einen oder mehrere Reste aus der Gruppe (C₁-C₄)Alkyl, (C₁-C₄)Haloalkyl, (C₁-C₄)Haloalkoxy, (C₁-C₄)Alkoxy und Halogen substituiert und
- R³: (C₃-C₆)Cycloalkyl, unsubstituiert oder durch einen oder mehrere Reste aus der Gruppe Halogen, (C₁-C₄)Alkoxy, (C₁-C₄)Alkylthio, [(C₁-C₄)Alkoxy]carbonyl, Phenyl und (C₁-C₄)Alkyl substituiert,
- R²: H oder (C₁-C₄)Alkyl und
- R³: Phenyl, das durch einen oder mehrere Reste aus der Gruppe (C₁-C₄)Haloalkyl substituiert ist, oder
- R²: (C₁-C₄)Alkyl und
- R³: CF₃ oder
- R², R³: gemeinsam mit dem C-Atom einen Ring mit 4-8 Ringatomen, der carbocyclisch ist oder heterocyclisch mit ein oder zwei Heteroatomen aus der Gruppe N, O und S ist und der unsubstituiert oder durch einen oder mehrere Reste aus der Gruppe (C₁-C₄)Alkyl, Halogen und Oxo substituiert ist, sowie
- R⁴: H oder (C₁-C₆)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, CN, Phenyl und (C₃-C₆)Cycloalkyl substituiert ist, oder (C₃-C₆)Alkenyl, (C₃-C₆)Alkinyl, wobei jeder der zwei letztgenannten Reste unsubstituiert oder durch ein oder mehrere Halogenatome substituiert ist, oder eine Gruppe der Formel 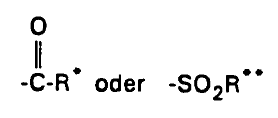
- R*: H, (C₁-C₈)Alkyl, (C₂-C₆)Alkenyl, (C₂-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, Phenoxy, [(C₁-C₄)Alkoxy]carbonyl, unsubstituiertes oder substituiertes Heterocyclyl und unsubstituiertes oder substituiertes Phenyl substituiert ist, oder unsubstituiertes oder substituiertes (C₃-C₆)Cycloalkyl, unsubstituiertes oder substituiertes Phenyl, unsubstituiertes oder substituiertes Heterocyclyl oder [(C₁-C₄)Alkoxy]carbonyl,
- R**: (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio und Phenyl substituiert ist, oder Phenyl, das unsubstituiert oder durch einen oder mehrere Reste aus der Gruppe Halogen, CN, NO₂, (C₁-C₄)Alkyl, (C₁-C₄)Haloalkyl und (C₁-C₄)Alkoxy substituiert ist,
- W: O oder S,
- X und Y: unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₃)Alkoxy und (C₁-C₄)Alkylthio substituiert ist, Mono- oder Di[(C₁-C₄)alkyl]amino, (C₃-C₆)-Cycloalkyl, (C₃-C₅)Alkenyl, (C₃-C₅)Alkenyloxy oder (C₃-C₅)Alkinyloxy und
- Z: CH oder N
bedeuten.

Von besonderem Interesse sind auch erfindungsgemäße Verbindungen der Formel (I) und deren Salze, worin
- R¹: (C₁-C₆)Alkyl, das unsubstituiert oder durch einen oder mehrere Reste aus der Gruppe Halogen und (C₁-C₄)Alkoxy substituiert ist, oder (C₃-C₄)Alkenyl oder (C₃-C₄)Alkinyl, oder Heterocyclyl mit 3 bis 6 Ringatomen und 1 oder 2 Heteroringatomen aus der Gruppe N und O, beispielsweise 3-Oxetanyl,
- R²: Phenyl, das durch einen oder mehrere Reste aus der Gruppe (C₁-C₄)Alkyl, (C₁-C₄)Alkoxy und Halogen substituiert ist, oder (C₃-C₄)Alkenyl und
- R³: (C₃-C₆)Cycloalkyl, das durch einen oder mehrere Reste aus der Gruppe Fluor, Brom, CN, NO₂, (C₁-C₄)Haloalkyl, (C₁-C₄)Alkoxy, (C₁-C₄)Alkylthio und [(C₁-C₄)-Alkoxy]carbonyl substituiert ist, oder
- R²: H oder (C₁-C₄)Alkyl und
- R³: Phenyl, das durch einen oder mehrere Reste aus der Gruppe (C₁-C₄)Haloalkyl, substituiert ist, oder
- R²: (C₁-C₄)Alkyl und
- R³: CF₃ oder
- R², R³: gemeinsam mit dem C-Atom einen Ring mit 4-8 Ringgliedern, der carbocyclisch oder heterocyclisch mit einem Heteroatom aus der Gruppe N, O und S ist und unsubstituiert oder ein- oder mehrfach durch (C₁-C₄)Alkyl, Halogen oder eine Oxogruppe substituiert ist, und
- R⁴: H, (C₁-C₄)Alkyl, das unsubstituiert oder durch einen oder mehrere Reste aus der Gruppe Halogen, (C₁-C₄)Alkoxy, (C₁-C₄)Alkylthio, [(C₁-C₄)Alkoxy]carbonyl und Phenyl substituiert ist, oder (C₃-C₄)Alkenyl oder (C₃-C₄)Alkinyl,
- R⁵: H, (C₁-C₄)Alkyl, (C₁-C₄)Haloalkyl, (C₁-C₄)Alkoxy oder Halogen,
- X und Y: unabhängig voneinander (C₁-C₄)Alkyl, (C₁-C₄)Alkoxy, wobei jeder der letztgenannten zwei Reste unsubstituiert oder durch einen oder mehrere Halogenatome substituiert ist, oder (C₁-C₄)Alkylthio, Halogen oder Mono- oder Di[(C₁-C₂)alkyl]amino und
- W: O
bedeuten.

Bevorzugte Verbindungen der Formel (I) oder deren Salze sind solche, worin
- R²: H oder (C₁-C₄)Alkyl und
- R³: Phenyl, das durch einen oder mehrere Reste aus der Gruppe (C₁-C₄)Haloalkyl substituiert ist, oder
- R² und R³: gemeinsam mit dem C-Atom einen Ring mit 5 oder 6 Ringgliedern, der gegebenenfalls ein Heteroatom aus der Gruppe N, O und S enthalten kann und unsubstituiert oder ein- oder mehrfach durch (C₁-C₂)Alkyl substituiert ist, sowie
- R⁴: H
- R⁵: H, (C₁-C₄)Alkyl oder Halogen,
- X: (C₁-C₂)Alkyl, (C₁-C₂)Alkoxy, (C₁-C₂)Alkylthio, (C₁-C₂)Haloalkyl oder (C₁-C₂)Haloalkoxy und
- Y: (C₁-C₂)Alkyl, (C₁-C₂)Alkoxy, Halogen, NHCH₃ oder N(CH₃)₂
bedeuten.

Besonders bevorzugte Verbindungen der Formel (I) oder deren Salze sind solche, worin
- R¹: Methyl oder Ethyl,
- R² und R³: gemeinsam mit dem C-Atom einen carbocyclischen Ring mit 5 oder 6 Ringgliedern, der unsubstituiert oder ein- oder mehrfach durch (C₁-C₂)Alkyl substituiert ist,
- R⁴: H und
- R⁵: H
bedeuten.

Gegenstand der vorliegenden Erfindung sind auch Verfahren zur Herstellung der Verbindungen der allgemeinen Formel (I) oder deren Salze, dadurch gekennzeichnet, daß man
a) eine Verbindung der Formel (II) 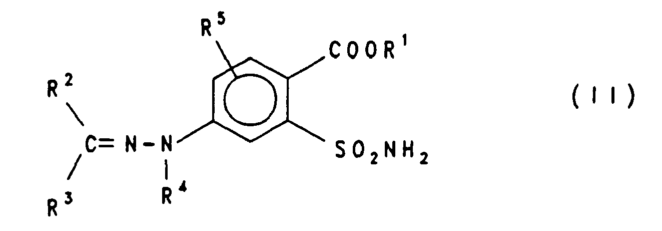 mit einem heterocyclischen Carbamat der Formel (III), 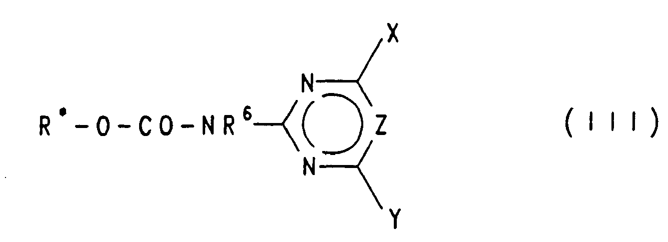 worin R* gegebenenfalls substituiertes Phenyl oder (C₁-C₄)Alkyl bedeutet, umsetzt oder
b) ein Sulfonylcarbamat der Formel (IV) 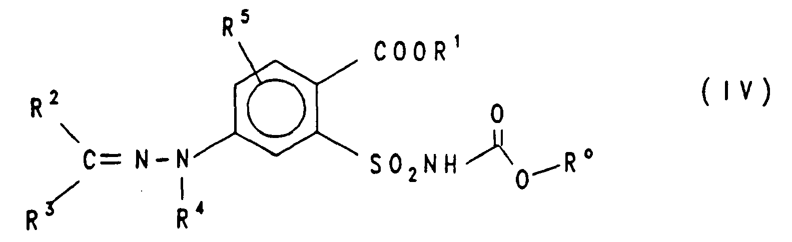 worin R^{o} unsubstituiertes oder substituiertes Phenyl oder (C₁-C₄)Alkyl bedeutet, mit einem Aminoheterocyclus der Formel (V) 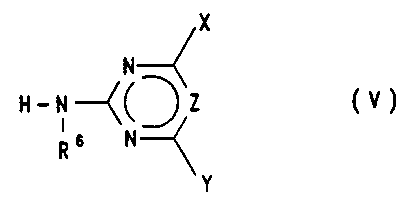 umsetzt oder
c) ein Sulfonylisocyanat der Formel (VI) 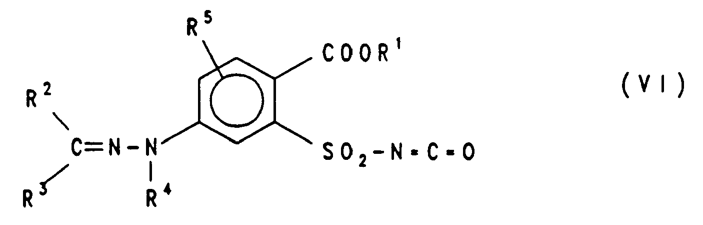 mit einem Aminoheterocyclus der Formel (V) (siehe Variante b) umsetzt oder
d) ein Sulfonamid der Formel (II) (siehe Variante a) mit einem (Thio)-Isocyanat der Formel (VII) 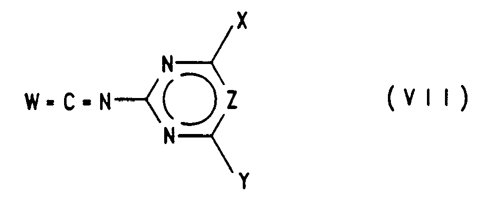 in Gegenwart einer Base umsetzt oder
e) einen Aminoheterocyclus der Formel (V) (siehe Variante a) zunächst basenkatalysiert mit einem Kohlensäureester, z.B. Diphenylcarbonat, umsetzt und das gebildete Intermediat in einer Eintopfreaktion mit einem Sulfonamid der Formel (II) (siehe Variante a) umsetzt,
wobei in den Formeln (II)-(VII) die Reste bzw. Gruppen R¹-R⁶, W, X, Y und Z wie in Formel (I) definiert sind und in Verfahrensvarianten a) bis c) und e) zunächst Verbindungen (I) mit W = O erhalten werden.

Die Umsetzung der Verbindungen der Formeln (II) und (III) erfolgt vorzugsweise basenkatalysiert in einem inerten organischen Lösungsmittel, wie z.B. Dichlormethan, Acetonitril, Dioxan oder THF (Tetrahydrofuran) bei Temperaturen zwischen 0°C, vorzugsweise 20°C, und dem Siedepunkt des Lösungsmittels. Als Base werden dabei beispielsweise organische Aminbasen, wie 1,8-Diazabicyclo[5.4.0]undec-7-en (DBU), insbesondere bei R^{o} = (subst.) Phenyl (vgl. EP-A-44807), oder Trimethylaluminium oder Triethylaluminium, letztere insbesondere bei R^{o} = Alkyl (vgl. EP-A-166 516) verwendet. Die jeweilige Base wird dabei beispielsweise im Bereich von 1 bis 3 Moläquivalenten, bezogen auf die Verbindung der Formel (II) eingesetzt. Die Sulfonamide (II) sind neue Verbindungen. Sie und ihre Herstellung sind ebenfalls Gegenstand dieser Erfindung.

Man erhält die Verbindungen der Formel (II) z.B. ausgehend von Verbindungen der Formel (VIII), 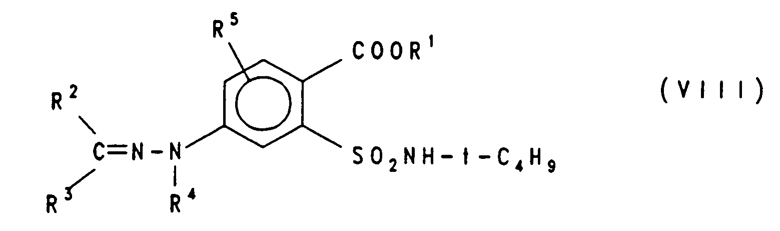 worin R¹-R⁵ wie in Formel (I) definiert sind, durch Umsetzung mit einer starken Säure (vgl. hierzu WO 89/10921).

Als starke Säuren kommen z.B. Mineralsäuren, wie H₂SO₄ oder HCl, oder starke organische Säuren, wie Trifluoressigsäure in Frage. Die Abspaltung der tert.-Butyl-Schutzgruppe erfolgt beispielsweise bei Temperaturen von -20°C bis zur jeweiligen Rückflußtemperatur des Reaktionsgemisches, vorzugsweise bei 0°C bis 40°C. Die Umsetzung kann in Substanz oder auch in einem inerten Solvens, wie z.B. Dichlormethan oder Trichlormethan, durchgeführt werden.

Die Verbindungen der Formel (VIII) werden z.B. aus geeigneten Hydrazinvorstufen durch Umsetzung mit geeigneten Elektrophilen, wie z.B. Aldehyden oder Ketonen erhalten. Geeignete Hydrazinvorstufen sind z.B. solche, worin R⁴ wie in Formel (I) definiert, insbesondere aber R⁴ = H bedeuten; zur Umsetzung der Hydrazinvorstufen mit Elektrophilen vgl.:
V. Lerch, J. König, Synthesis, 157 (1983),
Houben-Weyl, "Methoden der organischen Chemie", 4. Aufl., Bd. 10/2, S. 396 ff, S. 410 ff.

In einigen Fällen ist es möglich nach literaturbekannten Methoden im Anschluß an die Umsetzung mit Elektrophilen weitere Derivatisierungen vorzunehmen, z.B. Alkylierungen oder Acylierungen (vgl. Houben-Weyl, "Methoden der organischen Chemie", 4. Aufl. Bd. 10/2, S. 402 ff und S. 385).

Die Phenylhydrazine der Formel (VIIIa) sind zugänglich ausgehend von den Anilin-Derivaten (IX), 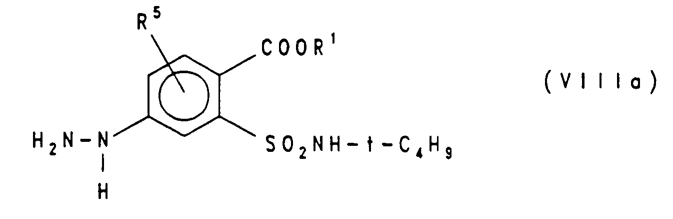 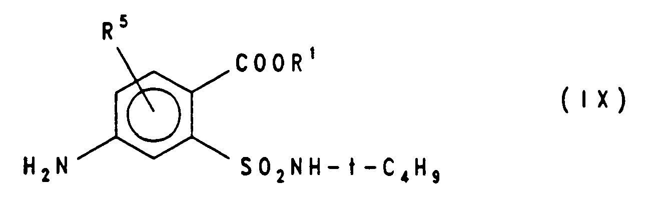 worin R¹ und R⁵ wie in Formel (I) definiert ist, durch Diazotierung und anschließende Reduktion. Geeignete Reduktionsmittel sind z.B. SnCl₂, SO₂ oder Natriumdithionit (vgl. Houben-Weyl, "Methoden der organischen Chemie", 4. Aufl. Bd. 10/2, S. 180 ff.; EP-A-562 575).

Die genannten Anilin-Derivate der Formel (IX) werden nach literaturbekannten Verfahren durch Reduktion der Nitrogruppe der Verbindungen (X), z.B. durch Hydrierung mit Wasserstoff in Gegenwart eines geeigneten Katalysators, wie Pd-C oder Raney-Nickel, oder durch Reduktion mit Eisen in essigsaurem Medium erhalten.
(vgl. hierzu: H. Berrie, G. T. Neuhold, F. S. Spring, J. Chem. Soc. (1952), 2042; M. Freifelder, "Catalytic Hydrogenation in Organic Synthesis: Procedures and Commentary", J. Wiley and Sons, New York (1978), Kap. 5).

Die aromatischen Sulfonamide der Formel (X) können aus den Sulfonsäuren der Formel (XI) gewonnen werden. 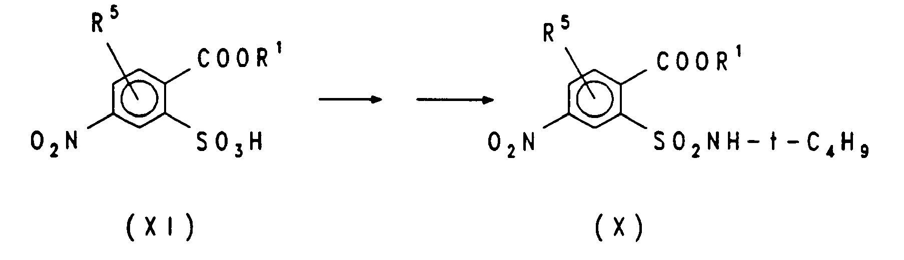 Zunächst erfolgt die Überführung der Sulfonsäuregruppe der Verbindungen (XI) in die Sulfochloride, z.B. nach Standardmethoden wie Umsetzung von Phosphoroxychlorid oder Thionylchlorid mit Kalium-Salzen der entsprechenden Sulfonsäuren in inerten Solventien wie Acetonitril und/oder Sulfolan oder in Substanz durch Erwärmen zum Rückfluß (vgl. Houben-Weyl-Klamann, "Methoden der organischen Chemie", 4. Aufl. Bd. E X1/2, S. 1067-1073, Thieme Verlag Stuttgart, 1985).

Die Sulfonamidbildung aus den Sulfochloriden mit tert.-Butylamin in Ethanol oder THF liefert die Verbindungen (X) in guten Ausbeuten (vgl. analoge Umsetzungen in WO 89/10921).

Die Sulfonsäuren der Formel (XI) sind aus der kommerziell erhältlichen 2-Methyl-5-nitrobenzolsulfonsäure darstellbar.

Durch Oxidation der Methylgruppe von 2-Methyl-5-nitrobenzolsulfonsäure durch Standardmethoden, wie z.B. die Umsetzung mit Kaliumpermanganat zur Carbonsäurefunktion sowie nachfolgende Veresterung wird der Substituent COOR¹ eingeführt. (vgl. hierzu: Houben-Weyl-Falbe: "Methoden der organischen Chemie", 4. Aufl. Bd. E V/1, Thieme Verlag Stuttgart, 1985, S. 199-202).

Alternativ hierzu sind die Zwischenprodukte der Formel (X) auch ausgehend von der kommerziell verfügbaren 2-Amino-5-nitrobenzoesäure nach folgendem Reaktionsschema zugänglich, 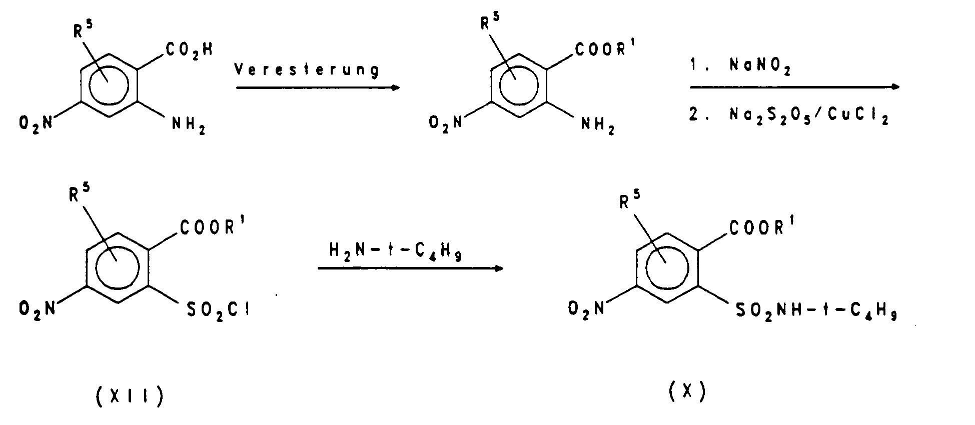 wobei alle Reaktionsschritte analog zu literaturbekannten Methoden durchgeführt werden können.

Die Verbindungen der Formel (II) lassen sich in analoger Weise auch unter Umgehung der t-Butyl-Schutzgruppe herstellen (vgl. EP 562 575). Dazu setzt man eine Verbindung der Formel (XII) statt mit t-Butylamin mit Ammoniak um und verfährt weiter wie für Verbindung (X) bzw. in EP 562 575 beschrieben.

Die Carbamate der Formel (III) können nach Methoden hergestellt werden, die in den südafrikanischen Patentanmeldungen 82/5671 und 82/5045 bzw. EP-A 70804 (US-A-4 480 101) oder RD 275056 beschrieben sind.

Die Umsetzung der Verbindungen (IV) mit den Aminoheterocyclen (V) führt man vorzugsweise in inerten, aprotischen Lösungsmitteln wie z.B. Dioxan, Acetonitril oder Tetrahydrofuran bei Temperaturen zwischen 0°C und der Siedetemperatur des Lösungsmittels durch. Die benötigten Ausgangsmaterialien (V) sind literaturbekannt oder können nach literaturbekannten Verfahren hergestellt werden. Die Phenylsulfonylcarbamate der Formel (IV) erhält man analog US-A-4 684 393 oder US-A-4 743 290.

Die Phenylsulfonylisocyanate der Formel (VI) lassen sich analog US-A-4 481 029 herstellen und mit den Aminoheterocyclen (V) umsetzen.

Die (Thio-)Isocyanate der Formel (VII) sind nach literaturbekannten Verfahren erhältlich (EP-A-232067, EP-A-166516). Die Umsetzung der (Thio)-Isocyanate (VII) mit Verbindungen (II) erfolgt bei -10°C bis 100°C, vorzugsweise 20 bis 100°C, in einem inerten aprotischen Lösungsmittel, wie z.B. Aceton oder Acetonitril, in Gegenwart einer geeigneten Base, z.B. N(C₂H₅)₃ oder K₂CO₃.

Die Umsetzung eines Aminoheterocyclus der Formel (V) mit Diphenylcarbonat und einem Sulfonamid der Formel (II) in einer Eintopfreaktion kann gemäß EP-A-562 575 durchgeführt werden.

Die Salze der Verbindungen der Formel (I) werden vorzugsweise in inerten polaren Lösungsmitteln wie z.B. Wasser, Methanol oder Aceton bei Temperaturen von 0-100°C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken-oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, lpomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono-und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden:
Alkylarylsulfonsaure Calzium-Salze wie
Ca-dodecylbenzolsulfonat oder nichtionische Emulgatoren wie
Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.
Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d. h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d. h. Ammoniumsulfamat; anilofos; asulam; atrazin; azimsulfurone (DPX-A8947); aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d. h. 2-Chlor-N,N-di-2-propenylacetamid; CDEC, d. h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyll-N-methyl-1H-pyrazole-4-carboxamid; endothal; EPTC; esprocarb; ethalfluralin; ethametsulfuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosaten; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d. h. N-[3-Chlor-4-(1-methylethyl)-phenyl]-2-methylpentanamid; naproanilide; napropamide; naptalam; NC 310, d. h. 4-(2,4-dichlorbenzoyl)-1-methyl-5-benzyloxypyrazot; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d. h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d. h. 2-[[7-[2-Chlor-4-(trifluor-methyl)-phenoxy]-2-naphthalenyl]-oxy]-propansäureund -methylester; sulfentrazon (FMC-97285, F-6285); sulfazuron; sulfometuronmethyl; sulfosate (ICI-A0224); TCA; tebutam (GCP-5544); tebuthiuron; terbacil; terbucarb; terbuchlor; terbumeton; terbuthylazine; terbutryn; TFH 450, d. h. N,N-Diethyl-3-[(2-ethyl-6-methylphenyl)-sulfonyl]-1H-1,2,4-triazol-1-carboxamid; thenylchlor (NSK-850); thiazafluron; thizopyr (Mon-13200); thidiazimin (SN-124085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d. h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### Beispiel A1

### 4-Amino-2-(N-tert-butylsulfamoyl)benzoesäuremethylester

Zu einem Gemisch aus 180 ml Essigsäure und 75 ml Wasser gibt man 50,0 g (0,158 mol) 2-(N-tert-Butylsulfamoyl)-4-nitrobenzoesäuremethylester (hergestellt nach DE-OS 4 236 902) und erwärmt auf 80°C. Man setzt 26,5 g (0,474 mol) Eisenpulver portionsweise so zu, daß die Temperatur nicht über 85°C ansteigt. Anschließend rührt man 4 h bei 80°C, fügt bei dieser Temperatur 85 ml 2N HCl zu und läßt auf 25°C abkühlen. Man filtriert ab und wäscht den Feststoff gründlich mit Wasser. Der Feststoff wird anschließend 3x mit je 250 ml Essigester heiß extrahiert. Die Essigesterphase dampft man ein, verreibt den Rückstand mit Diisopropylether und trocknet. Man erhält 37,7 g (83% d.Th.) 4-Amino-2-(N-tert-butylsulfamoyl)benzoesäuremethylester vom Schmp. 198-199°C.

### Beispiel A2

### 2-(N-tert-Butylsulfamoyl)-4-hydrazinobenzoesäuremethylester

25,0 g (0,087 mol) 4-Amino-2-(N-tert-butylsulfamoyl)benzoesäuremethylester werden in einem Gemisch aus 150 ml konz. HCl und 130 ml Wasser suspendiert. Bei 0-5°C tropft man eine Lösung von 7,8 g (0,114 mol) Natriumnitrit zu. Man filtriert kalt von wenig ungelösten Bestandteilen ab und läßt die noch kalte Diazoniumsalzlösung bei 0°C in eine Suspension von 55,0 g (0,244 mol) SnCl₂•2H₂O in 55 ml konz. HCl langsam abfließen. Man rührt 30 Min. nach und läßt anschließend 15 h unter Eiskühlung stehen. Durch Zugabe von 6 N NaOH neutralisiert man zunächst die Hauptmenge HCl und bringt anschließend mit festen NaHCO₃ auf ca. pH 6. Man extrahiert mehrfach mit Essigester, trocknet und entfernt das Lösungsmittel im Vakuum. Nach Verreiben mit Diisopropylether und Trocknen erhält man 18,3 g (70% d.Th.) 2-(N-tert-Butylsulfamoyl)-4-hydrazinobenzoesäuremethylester vom Schmp. 133-136°C.

### Beispiel A3

### 2-(N-tert-Butylsulfamoyl)-4-cyclopentylidenhydrazinobenzoesäuremethylester

Zu 2,0 g (0,007 mol) 2-(N-tert-Butylsulfamoyl)-4-hydrazinobenzoesäure-methylester in 100 ml Methanol gibt man 2,8 g (0,032 mol) Cyclopentanon und rührt 3 h bei 65°C nach. Man dampft ein und verreibt den Rückstand mit wenig Methanol. Nach Absaugen und Trocknen erhält man 1,3 g (51% d.TH.) 2-(N-tert-Butylsulfamoyl)-4-cyclopentylidenhydrazinobenzoesäuremethylester;
¹H-NMR (d₆-DMSO): δ = 1,17 (s, 9H, t-C₄H₉); 1,65 - 1,85 (m, 4H, 3,4-CH₂); 2,30 - 2,45 (m, 4H, 2,5-CH₂); 3,81 (s, 3H, OCH₃); 6,84 (s, 1H, SO₂NH); 7,20 (dd, 1H, ArH); 7,71 (d, 1 H, ArH); 7,72 (d, 1 H, ArH); 9,55 (s, 1 H, N-NH).

### Beispiel A4

### 4-Cyclopentylidenhydrazino-2-sulfamoylbenzoesäuremethylester

3,9 g (10,6 mmol) 2-(N-tert-Butylsulfamoyl)-4-cyclopentylidenhydrazino-benzoesäuremethylester werden in 40 ml Trifluoressigsäure 2 h bei 25°C gerührt. Man dampft ein und verreibt den Rückstand mit Diethylether. Nach Absaugen und Trocknen erhält man 3,0 g (91 % d.Th.) 4-Cyclopentyliden-hydrazino-2-sulfamoylbenzoesäuremethylester vom Schmp.: 227-229°C (Zers.).

### Beispiel A5

### 4-Cyclopentylidenhydrazino-2-[3-(4,6-dimethoxypyrimidin-2-yl)ureidosulfonyl]-benzoesäuremethylester (siehe Tabelle 1, Beispiel Nr. 40)

1,5 g (4,8 mmol) 4-Cyclopentylidenhydrazino-2-sulfamoylbenzoesäuremethylester und 1,6 g (5,8 mmol) Phenyl-N-(4,6-dimethoxypyrimidin-2-yl)carbamat werden in 20 ml Acetonitril vorgelegt. Bei 0°C tropft man 1,6 g (10,6 mmol) 1,8-Diazabicyclo[5,4,0]undec-7-en (DBU) zu und rührt 2 h bei dieser Temperatur. Man gießt in Wasser und stellt den pH-Wert mit 2N Salzsäure auf 2-3 ein. Die Wasserphase wird dreimal mit CH₂Cl₂ extrahiert. Nach Waschen der Methylenchloridphase mit 2N Salzsäure und Wasser trocknet man und dampft ein. Der Rückstand wird mit Diisopropylether verrieben. Nach Absaugen und Trocknen erhält man 1,55 g (65 % d.Th.) 4-Cyclopentylidenhydrazino-2-[3-(4,6-dimethoxypyrimidin-2-yl)-ureidosulfonyl]benzoesäuremethylester vom Schmp. 168-170°C (Zers.).

Die in den Tabellen 1-3 beschriebenen Verbindungen erhält man analog zu den Beispielen A1-A5. In der Tabellen bedeuten:
- Bsp. =: Beispiel oder Beispielnummer
- Fp. =: Festpunkt (Schmelzpunkt) in °C
- Me =: Methyl
- Et =: Ethyl
- Pr =: n-Propyl
- i-Pr =: Isopropyl
- c-Pr =: Cyclopropyl
- Bu =: n-Butyl
- Ph =: Phenyl
- (Z) =: (Z.) = (Zers.) = Schmelzpunkt unter Zersetzung

**Tabelle 1: Verbindungen der Formel (la)**

| | | | | | | | | |
|---|---|---|---|---|---|---|---|---|
| 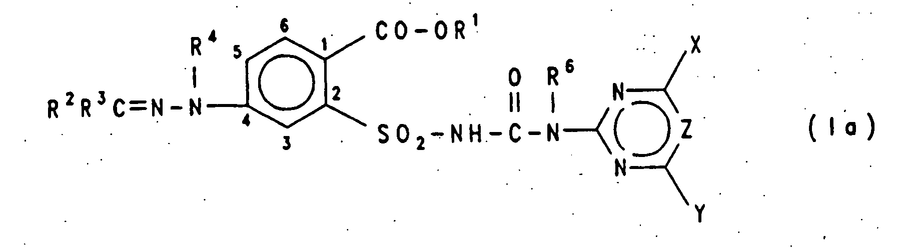 | | | | | | | | |

| Bsp. Nr. | R¹ | CR²R³ | R⁴ | R⁶ | X | Y | Z | Fp. [°C] |
|---|---|---|---|---|---|---|---|---|
| 12 | " | 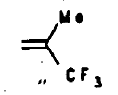 | " | H | " | OMe | CH | 147-54 (Z) |
| 13 | " | " | " | " | " | Me | N | |
| 20 | " | 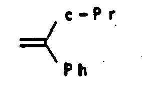 | " | " | " | OMe | CH | 124-26 (Z.) |
| 21 | " | " | " | | " | Me | N | |
| 26 | " | 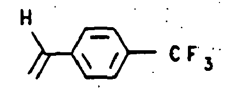 | " | " | " | OMe | CH | 194-96 (Z.) |
| 27 | " | " | " | " | " | Me | N | |
| 28 | " | 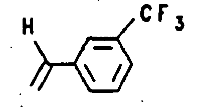 | " | " | " | OMe | CH | 219-21 (Z.) |
| 29 | " | " | " | H | " | Me | N | |
| 40 | " | 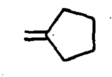 | " | H | " | OMe | CH | 168-70 (Z.) |
| 41 | " | " | " | " | " | Me | CH | |
| 42 | " | " | " | " | " | OMe | N | 187-90 (Z) |
| 43 | " | " | " | " | " | Me | N | 173-77 (Z) |
| 44 | " | " | " | H | " | Cl | CH | 183-86 (Z) |
| 45 | " | " | " | " | Me | Me | CH | |
| 46 | " | " | " | " | OCH₂CF₃ | NMe₂ | N | |
| 47 | " | " | " | " | CF₃ | OMe | N | |
| 48 | " | " | " | " | OCHF₂ | OCHF₂ | CH | |
| 49 | " | " | " | Me | OMe | OMe | CH | |
| 50 | " | " | " | " | " | Me | N | |
| 51 | " | 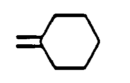 | " | H | " | OMe | CH | |
| 52 | " | " | " | " | " | Me | CH | |
| 53 | " | " | " | " | " | OMe | N | |
| 54 | " | " | " | " | " | Me | N | |
| 55 | " | " | " | " | " | Cl | CH | |
| 56 | " | " | " | " | Me | Me | CH | |
| 57 | " | " | " | " | OCH₂CF₃ | NMe₂ | N | |
| 58 | " | " | " | " | CF₃ | OMe | N | |
| 59 | " | " | " | " | OCHF₂ | OCHF₂ | CH | |
| 60 | " | " | " | Me | " | OMe | CH | |
| 61 | " | " | " | " | " | Me | N | |
| 62 | " | 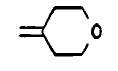 | " | H | OMe | OMe | CH | 155-57 (Z.) |
| 63 | " | " | " | " | " | Me | N | |
| 64 | " | 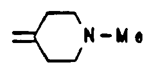 | " | " | " | OMe | CH | |
| 65 | " | " | " | " | " | Me | N | |
| 66 | " | 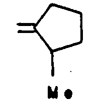 | " | " | " | OMe | CH | |
| 67 | " | " | " | " | " | Me | N | |
| 68 | " | 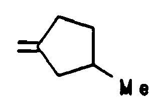 | " | " | " | OMe | CH | |
| 69 | " | " | " | " | " | Me | N | |
| 70 | " | 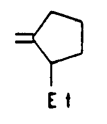 | " | " | " | OMe | CH | |
| 71 | " | Et | " | " | " | Me | N | |
| 72 | " | 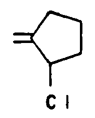 | " | " | " | OMe | CH | |
| 73 | " | " | " | " | " | Me | N | |
| 74 | Et | 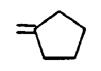 | " | " | " | OMe | CH | |
| 75 | | " | " | " | " | Me | N | |
| 76 | Pr | " | " | " | " | OMe | CH | |
| 77 | " | " | " | " | " | Me | N | |
| 78 | i-Pr | " | " | " | " | OMe | CH | |
| 79 | " | " | " | " | " | Me | N | |
| 80 | Oxetan-3-yl | " | " | " | " | OMe | CH | |
| 81 | " | " | " | " | " | Me | N | |
| 82 | Et | " | " | " | " | OMe | CH | |
| 83 | " | 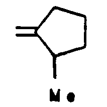 | " | " | " | Me | N | |
| 84 | " | " | " | " | " | OMe | CH | |
| 85 | " | " | " | " | " | Me | N | |
| 86 | " | 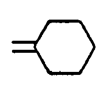 | " | " | " | OMe | CH | |
| 87 | " | " | " | " | " | Me | N | |
| 88 | " | 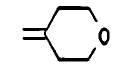 | " | " | " | OMe | CH | |
| 89 | " | " | " | " | " | Me | N | |

**Tabelle 2: Verbindungen der Formel (Ib)**

| | | | | | | |
|---|---|---|---|---|---|---|
| 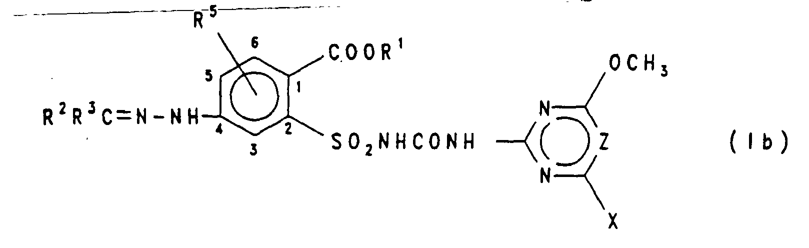 | | | | | | |

| Bsp. | R¹ | CR²R³ | R⁵ | X | Z | Fp |
|---|---|---|---|---|---|---|
| 2/1 | Me | 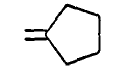 | 3-Me | OMe | CH | |
| 2/2 | " | " | 6-Me | " | " | |
| 2/3 | " | " | 3-Cl | " | " | |
| 2/4 | " | " | 5-Cl | " | " | |
| 2/5 | " | " | 6-Cl | " | " | |
| 2/6 | Et | " | 3-Me | " | " | |
| 2/7 | " | " | 6-Me | " | " | |
| 2/8 | " | " | 3-Cl | " | " | |
| 2/9 | " | " | 5-Cl | " | " | |
| 2/10 | " | " | 6-CI | " | " | |
| 2/11 | Me | | 3-Me | Me | N | |
| 2/12 | " | " | 6-Me | " | " | |
| 2/13 | " | | 3-CI | " | " | |
| 2/14 | " | | 5-Cl | " | " | |
| 2/15 | " | " | 6-0 | " | " | |
| 2/16 | Et | " | 3-Me | " | " | |
| 2/17 | " | " | 6-Me | " | " | |
| 2/18 | " | " | 3-Cl | " | " | |
| 2/19 | " | " | 5-Cl | " | " | |
| 2/20 | " | " | 6-Cl | " | " | |
| 2/21 | Me | " | 5-OMe | OMe | CH | |
| 2/22 | " | " | 5-CF₃ | " | " | |

**Tabelle 3: Verbindungen (Salze) der Formel (Ic)**

| | | | | | | | | |
|---|---|---|---|---|---|---|---|---|
| 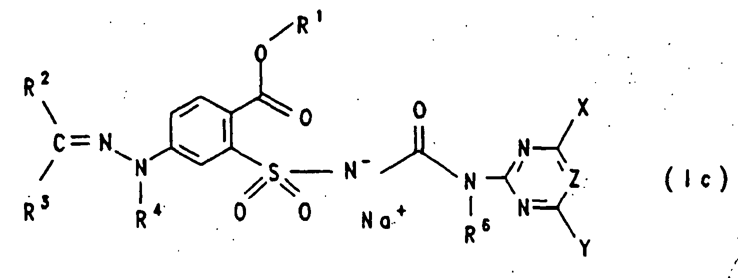 | | | | | | | | |

| Bsp. | R¹ | CR²R³ | R⁴ | R⁶ | X | Y | Z | Fp |
|---|---|---|---|---|---|---|---|---|
| 3/3 | " | 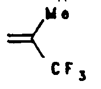 | " | " | " | OMe | CH | 225-228 (Z) |
| 3/4 | " | " | " | " | " | Me | N | |
| 3/5 | " | 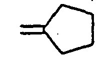 | " | " | " | OMe | CH | 196-200 (Z) |
| 3/6 | " | " | " | " | " | Me | N | 246-249 (Z) |
| 3/7 | " | " | " | " | " | " | " | CH |
| 3/8 | " | " | " | " | " | OMe N | | 214-217 (Z) |
| 3/9 | " | | " | " | " | Cl | CH | |
| 3/10 | " | " | " | " | Me | Me | CH | |
| 3/11 | " | " | " | " | OCH₂CF₃ | NMe₂ | N | |
| 3/12 | " | " | " | " | CF₃ | OMe | N | |
| 3/13 | " | " | " | " | OCHF₂ | OCHF₂ | CH | |
| 3/15 | " | " | " | Me | OMe | OMe | CH | |
| 3/16 | " | " | " | " | " | Me | N | |
| 3/17 | " | 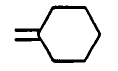 | " | H | " | OMe | CH | |
| 3/18 | " | " | " | " | " | Me | N | |
| 3/19 | " | 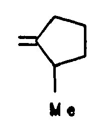 | " | " | " | OMe | CH | |
| 3120 | " | " | " | " | " | Me | N | |
| 3/21 | Et | 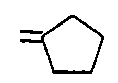 | " | " | " | OMe | CH | |
| 3/22 | " | " | " | " | " | Me | N | |

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (^{®}Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
75 Gewichtsteile einer Verbindung der Formel (I),

| | | |
|---|---|---|
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
25 Gewichtsteile einer Verbindung der Formel (I),

| | | |
|---|---|---|
| 5 | " | 2,2'-dinaphthylmethan-6,6'-disulfonsaures Natrium |
| 2 | " | oleoylmethyltaurinsaures Natrium, |
| 1 | Gewichtsteil | Polyvinylalkohol, |
| 17 | Gewichtsteile | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Beispiele Nr. 12, 20, 26, 28, 30, 40, 42, 43, 44, 62, 3/3 3/5, 3/6 und 3/8 (s. Tabellen 1 bis 3) sehr gute herbizide Wirkung gegen Schadpflanzen wie Sinapis alba, Chrysanthemum segetum, Avena sativa, Stellaria media, Echinochloa crus-galli, Lolium multiflorum, Setaria spp., Abutilon theophrasti, Amaranthus retroflexus und Panicum miliaceum im Vorauflaufverfahren bei einer Aufwandmenge von 0,1 kg und weniger Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt, wobei die als Spritzpulver bzw. als Emulsionskonzentrate formulierten erfindungsgemäßen Verbindungen in verschiedenen Dosierungen mit einer Wasseraufwandmenge von umgerechnet 600 bis 800 I/ha auf die grünen Pflanzenteile gesprüht werden. Nach ca. 3 bis 4 Wochen Standzeit der Versuchspflanzen im Gewächshaus unter optimalen Wachstumsbedingungen wird die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Beispiele Nr. 12, 20, 26, 28, 40, 42, 43, 44, 62, 3/3, 3/5, 3/6 und 3/8 (s. Tabellen 1 bis 3) sehr gute herbizide Wirkung gegen Schadpflanzen wie Sinapis alba, Echinochloa crus-galli, Lolium multiflorum, Chrysanthemum segetum, Setaria spp., Abutilon theophrasti, Amaranthus retroflexus, Panicum miliaceum und Avena sativa im Nachauflaufverfahren bei einer Aufwandmenge von 0,1 kg und weniger Aktivsubstanz pro Hektar.

### 3. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt lassen. Einige Substanzen schonen darüber hinaus auch Gramineen-Kulturen wie z.B. Gerste, Weizen, Roggen, Sorghum-Hirsen, Mais oder Reis. Die Verbindungen der Formel (I) zeigen teilweise eine hohe Selektivität und eignen sich deshalb zur Bekämpfung von unerwünschten Pflanzenwuchs in landwirtschaftlichen Kulturen.

## Patentansprüche

1. Verbindungen der Formel (I) oder deren Salze, 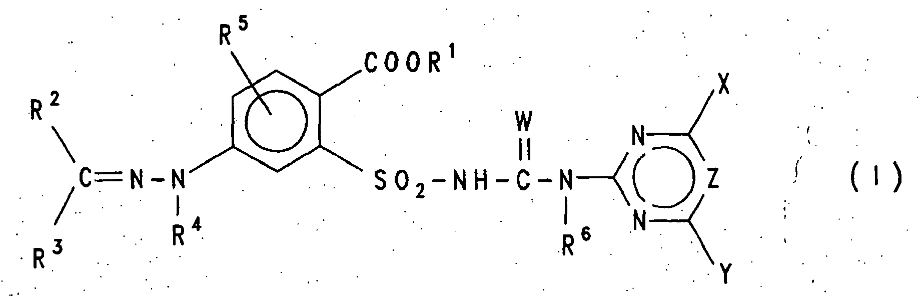 worin
R¹ H oder einen Kohlenwasserstoffrest, der unsubstituiert oder substituiert ist, oder einen Heterocyclylrest, der unsubstituiert oder substituiert ist,
R² Phenyl, unsubstituiert oder substituiert, und
R³ (C₃-C₆)Cycloalkyl, unsubstituiert oder substituiert, oder
R² Wasserstoff oder (C₁-C₄)Alkyl und
R³ Phenyl, das durch einen oder mehrere Reste aus der Gruppe (C₁-C₄)Haloalkyl, substituiert ist, oder
R² (C₁-C₄)Alkyl und
R³ CF₃ oder
R², R³ gemeinsam mit dem Methylen-C-atom einen Cycloalkylen- oder Heterocyclylenrest mit 4 bis 8 Ringatomen, der unsubstituiert oder substituiert ist, sowie
R⁴ H, einen unsubstituierten oder substituierten, aliphatischen Kohlenwasserstoffrest mit 1 bis 6 C-Atomen im Kohlenwasserstoffteil und ein oder mehreren Substituenten, wobei die Substituenten aus der Gruppe Halogen, (C₁-C₄)Alkoxy, (C₁-C₄)Alkylthio, (C₁-C₄)Alkylsulfonyl, [(C₁-C₄)Alkyl]carbonyl, [(C₁-C₄)-Alkoxy]cärbonyl, CN, substituiertes und unsubstituiertes Phenyl und (C₃-C₆)Cycloalkyl ausgewählt sind, oder (C₂-C₆)Alkenyl oder (C₂-C₆)Alkinyl oder einen Acylrest,
R⁵ H, Halogen, NO₂, CN, (C₁-C₄)Alkyl, (C₁-C₄)Alkoxy, [(C₁-C₄)Alkyl]-carbonyl oder[(C₁-C₄)Alkoxy]carbonyl, wobei jeder der letztgenannten vier Reste unsubstituiert oder im Alkylteil durch ein oder mehrere Halogenatome substituiert ist,
R⁶ H oder (C₁-C₄)Alkyl,
W ein Sauerstoff- oder Schwefelatom,
X, Y unabhängig voneinander H, Halogen, (C₁-C₄)Alkyl, (C₁-C₄)Alkoxy, (C₁-C₄)Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Mono- oder Di-[(C₁-C₄)alkyl]-amino, (C₃-C₆)Cycloalkyl, (C₂-C₅)Alkenyl, (C₂-C₅)Alkinyl, (C₂-C₅)-Alkenyloxy oder (C₂-C₅)Alkinyloxy und
Z CH oder N
bedeuten.

2. Verbindungen und deren Salze nach Anspruch 1, **dadurch gekennzeichnet, daß**
R¹ H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio und [(C₁-C₄)Alkoxy]-carbonyl substituiert ist, oder (C₃-C₆)Cycloalkyl, (C₃-C₆)Cycloalkyl(C₁-C₃)alkyl, Heterocyclyl mit 3 bis 6 Ringatomen oder Heterocyclyl-(C₁-C₃)alkyl mit 3 bis 6 Ringatomen, wobei jeder der letztgenannten 4 Reste unsubstituiert oder durch einen oder mehrere Reste aus der Gruppe Halogen, (C₁-C₄)Alkyl und (C₁-C₄)Alkoxy substituiert ist,
R² Phenyl, unsubstituiert oder durch einen oder mehrere Reste aus der Gruppe (C₁-C₄)Alkyl, (C₁-C₄)Haloalkyl, (C₁-C₄)Haloalkoxy, (C₁-C₄)Alkoxy und Halogen substituiert, und
R³ (C₃-C₆)Cycloalkyl, unsubstituiert oder durch einen oder mehrere Reste aus der Gruppe Halogen, (C₁-C₄)Alkoxy, (C₁-C₄)Alkylthlo, [(C₁-C₄)Alkoxy]carbonyl, Phenyl und (C₁-C₄)Alkyl substituiert oder
R² H oder (C₁-C₄)Alkyl und
R³ Phenyl, das durch einen oder mehrere Reste aus der Gruppe (C₁-C₄)Haloalkyl, substituiert ist, oder
R² (C₁-C₄)Alkyl und
R³ CF₃ oder
R², R³ gemeinsam mit dem C-Atom einen Ring mit 4-8 Ringatomen, der carbocyclisch ist oder heterocyclisch mit ein oder zwei Heteroatomen aus der Gruppe N, O und S ist und der unsubstituiert oder durch einen oder mehrere Reste aus der Gruppe (C₁-C₄)Alkyl, Halogen und Oxo substituiert ist, sowie
R⁴ H oder (C₁-C₆)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, CN, Phenyl und (C₃-C₆)Cycloalkyl substituiert ist, oder (C₃-C₆)Alkenyl, (C₃-C₆)Alkinyl, wobei jeder der zwei letztgenannten Reste unsubstituiert oder durch ein oder mehrere Halogenatome substituiert ist, oder eine Gruppe der Formel 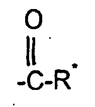 oder -SO₂R**
R⁺ H, (C₁-C₈)Alkyl, (C₂-C₆)Alkenyl, (C₂-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, Phenoxy, [(C₁-C₄)Alkoxy]carbonyl, unsubstituiertes oder substituiertes Heterocyclyl und unsubstituiertes oder substituiertes Phenyl substituiert ist, oder unsubstituiertes oder substituiertes (C₃-C₆)Cycloalkyl, unsubstituiertes oder substituiertes Phenyl, unsubstituiertes oder substituiertes Heterocyclyl oder [(C₁-C₄)Alkoxy]carbonyl,
R** (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio und Phenyl substituiert ist, oder Phenyl, das unsubstituiert oder durch einen oder mehrere Reste aus der Gruppe Halogen, CN, NO₂, (C₁-C₄)Alkyl, (C₁-C₄)Haloalkyl und (C₁-C₄)Alkoxy substituiert ist,
W O oder S,
X und Y unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₃)Alkoxy und (C₁-C₄)Alkylthio substituiert ist, Mono- oder Di[(C₁-C₄)alkyl]amino, (C₃-C₆)-Cycloalkyl, (C₃-C₅)Alkenyl, (C₃-C₅)Alkenyloxy oder (C₃-C₅)Alkinyloxy und
Z CH oder N
bedeuten.

3. Verbindungen und deren Salze nach Anspruch 1 oder 2, **dadurch gekennzeichnet, daß**
R¹ (C₁-C₆)Alkyl, das unsubstituiert oder durch einen oder mehrere Reste aus der Gruppe Halogen und (C₁-C₄)Alkoxy substituiert ist, oder (C₃-C₄)Alkenyl oder (C₃-C₄)Alkinyl, oder Heterocyclyl mit 3 bis 6 Ringatomen und 1 oder 2 Heteroringatomen aus der Gruppe N und O,
R² Phenyl, das durch einen oder mehrere Reste aus der Gruppe (C₁-C₄)Alkyl, (C₁-C₄)Alkoxy und Halogen substituiert ist, und
R³ (C₃-C₆)Cycloalkyl, das durch einen oder mehrere Reste aus der Gruppe Fluor, Brom, CN, NO₂, (C₁-C₄)Haloalkyl, (C₁-C₄)Alkoxy, (C₁-C₄)Alkylthio und [(C₁-C₄)- Alkoxy]carbonyl substituiert ist, oder
R² H oder (C₁-C₄)Alkyl und
R³ Phenyl, das durch einen oder mehrere Reste aus der Gruppe (C₁-C₄)Haloalkyl, substituiert ist, oder
R² (C₁-C₄)Alkyl und
R³ CF₃ oder
R², R³ gemeinsam mit dem C-Atom einen Ring mit 4-8 Ringgliedern, der carbocyclisch oder heterocyclisch mit einem Heteroatom aus der Gruppe N, O und S ist und unsubstituiert oder ein- oder mehrfach durch (C₁-C₄)Alkyl, Halogen oder eine Oxogruppe substituiert ist, und
R⁴ H, (C₁-C₄)Alkyl, das unsubstituiert oder durch einen oder mehrere Reste aus der Gruppe Halogen, (C₁-C₄)Alkoxy, (C₁-C₄)Alkylthio, [(C₁-C₄)Alkoxy]carbonyl und Phenyl substituiert ist, oder (C₃-C₄)Alkenyl oder (C₃-C₄)Alkinyl,
R⁵ H, (C₁-C₄)Alkyl, (C₁-C₄)Haloalkyl, (C₁-C₄)Alkoxy oder Halogen,
X und Y unabhängig voneinander (C₁-C₄)Alkyl, (C₁-C₄)Alkoxy, wobei jeder der letztgenannten zwei Reste unsubstituiert oder durch einen oder mehrere Halogenatome substituiert ist, oder (C₁-C₄)Alkylthio, Halogen oder Mono- oder Di[(C₁-C₂)alkyl]amino und
W O bedeuten.

4. Verbindungen und deren Salze nach einem der Ansprüche 1 bis 3, **dadurch gekennzeichnet, daß**
R² H oder (C₁-C₄)Alkyl und
R³ Phenyl, das durch einen oder mehrere Reste aus der Gruppe (C₁-C₄)Haloalkyl substituiert ist, oder
R² und R³ gemeinsam mit dem C-Atom einen Ring mit 5 oder 6 Ringgliedern, der gegebenenfalls ein Heteroatom aus der Gruppe N, O und S enthalten kann und unsubstituiert oder ein- oder mehrfach durch (C₁-C₂)Alkyl substituiert ist, sowie
R⁴ H,
R⁵ H, (C₁-C₄)Alkyl oder Halogen,
X (C₁-C₂)Alkyl, (C₁-C₂)Alkoxy, (C₁-C₂)Alkylthio, (C₁-C₂)Haloalkyl oder (C₁-C₂)Haloalkoxy und
Y (C₁-C₂)Alkyl, (C₁-C₂)Alkoxy, Halogen, NHCH₃ oder N(CH₃)₂
bedeuten.

5. Verfahren zur Herstellung von Verbindungen der Formel (I) oder deren Salzen nach Anspruch 1, **dadurch gekennzeichnet, daß** man
a) eine Verbindung der Formel (II) 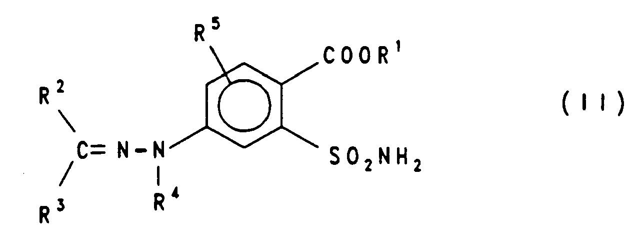 mit einem heterocyclischen Carbamat der Formel (III), 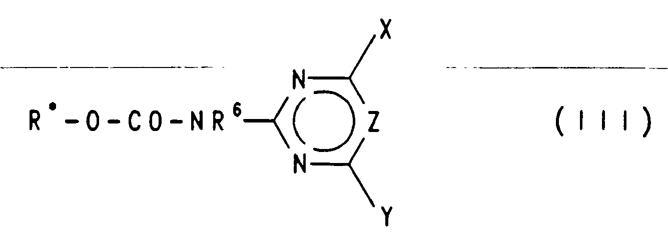 worin R* gegebenenfalls substituiertes Phenyl oder (C₁-C₄)Alkyl bedeutet, umsetzt oder
b) ein Sulfonylcarbamat der Formel (IV) 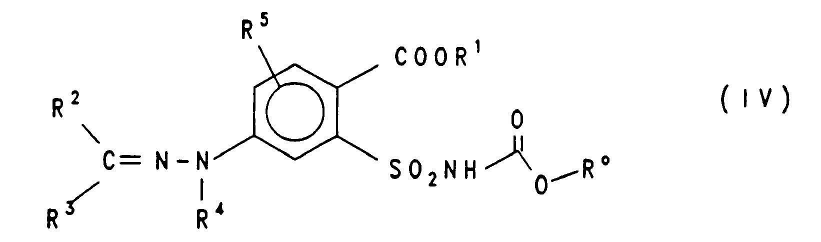 worin R^{o} unsubstituiert oder substituiertes Phenyl oder (C₁-C₄)Alkyl bedeutet, mit einem Aminoheterocyclus der Formel (V) 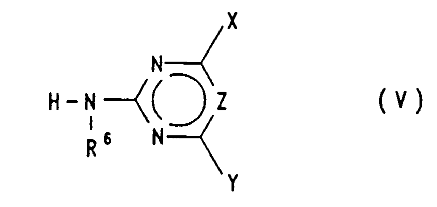 umsetzt oder
c) ein Sulfonylisocyanat der Formel (VI) 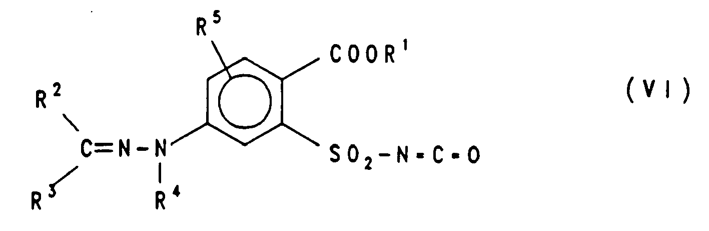 mit einem Aminoheterocyclus der Formel (V) umsetzt oder
d) ein Sulfonamid der Formel (II) mit einem (Thio)-Isocyanat der Formel (VII) 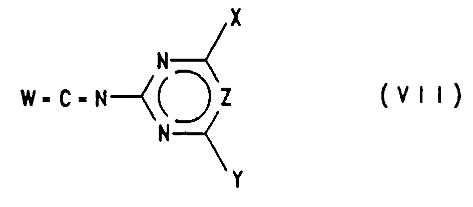 in Gegenwart einer Base umsetzt oder
e) einen Aminoheterocyclus der Formel (V) zunächst basenkatalysiert mit einem Kohlensäureester umsetzt und das gebildete Intermediat in einer Eintopfreaktion mit einem Sulfonamid der Formel (II) umsetzt,
wobei in den Formeln (II)-(VII) die Reste bzw. Gruppen R¹-R⁶, W, X, Y und Z wie in Formel (I) definiert sind und in Verfahrensvarianten a) bis c) und e) zunächst Verbindungen (I) mit W = O erhalten werden.

6. Herbizides oder pflanzenwachstumsregulierende Mittel, **dadurch gekennzeichnet, daß** es mindestens eine Verbindung der Formel (I) oder deren Salz nach einem der Ansprüche 1 bis 4 und im Pflanzenschutz übliche Formulierungshilfsmittel enthält.

7. Verfahren zur Bekämpfung von Schadpflanzen oder zur Wachstumsregulierung von Pflanzen, **dadurch gekennzeichnet, daß** man eine wirksame Menge von mindestens einer Verbindung der Formel (I) oder deren Salz nach einem der Ansprüche 1 bis 4 auf die Schadpflanzen bzw. Pflanzen, deren Pflanzensamen oder die Fläche, auf der sie wachsen, appliziert.

8. Verwendung der Verbindungen der Formel (I) oder deren Salze nach einem der Ansprüche 1 bis 4 als Herbizide oder Pflanzenwachstumsregulatoren.

9. Verbindungen der Formel (11), 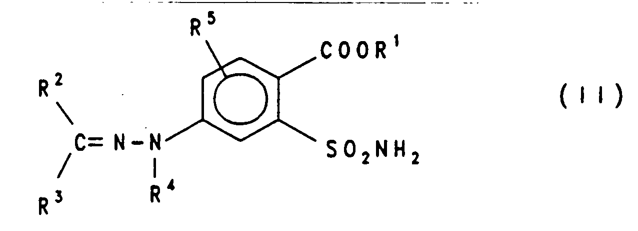 worin R¹ bis R⁵ wie in Formel (I) nach einem der Ansprüche 1 bis 4 definiert sind.

## Claims

1. A compound of the formula (I) or salt thereof 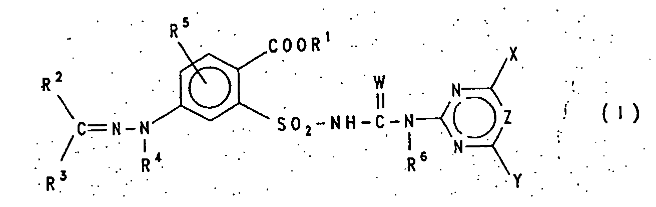 in which
R¹ is H or a hydrocarbon radical, which is unsubstituted or substituted, or a heterocyclyl radical, which is unsubstituted or substituted,
R² is phenyl which is unsubstituted or substituted, or
R³ is (C₃-C₆)cycloalkyl, which is unsubstituted or substituted, or
R² is hydrogen or (C₁-C₄)alkyl and
R³ is phenyl, which is substituted by one or more radicals from the group consisting of (C₁-C₄)-haloalkyl, or
R² is (C₁-C₄)alkyl and
R³ is CF₃, or
R² and R³, together with the methylene carbon atom, are a cycloalkylene or heterocyclylene radical having 4 to 8 ring atoms, which is unsubstituted or substituted, and
R⁴ is H, an unsubstituted or substituted aliphatic hydrocarbon radical having 1 to 6 carbon atoms in the hydrocarbon part and one or more substituents, where the substituents are selected from the group consisting of halogen, (C₁-C₄)-alkoxy, (C₁-C₄) alkylthio, (C₁-C₄)alkylsulfonyl, [(C₁-C₄) alkyl] carbonyl, [(C₁-C₄) -alkoxy] carbonyl, CN, substituted and unsubstituted phenyl and (C₃-C₆)cycloalkyl, or (C₂-C₆)alkenyl or (C₂-C₆)-alkynyl or an acyl radical,
R⁵ is H, halogen, NO₂, CN, (C₁-C₄)alkyl, (C₁-C₄)-alkoxy, [(C₁-C₄)alkyl] carbonyl or [(C₁-C₄)-alkoxy]carbonyl, where each of the last four radicals mentioned is unsubstituted or substituted by one or more halogen atoms in the alkyl part,
R⁶ is H or (C₁-C₄)alkyl,
W is an oxygen or sulfur atom,
X and Y independently of one another are H, halogen, (C₁-C₄) alkyl, (C₁-C₄) alkoxy or (C₁-C₄)-alkylthio, where each of the last 3 radicals mentioned is unsubstituted or substituted by one or more radicals from the group consisting of halogen, (C₁-C₄) alkoxy and (C₁-C₄) alkylthio, or mono- or di- [(C₁-C₄)alkyl]-amino, (C₃-C₆)cycloalkyl, (C₂-C₅)alkenyl, (C₂-C₅)alkynyl, (C₂-C₅)-alkenyloxy or (C₂-C₅)alkynyloxy and
Z is CH or N.

2. A compound or salt thereof as claimed in claim 1, in which
R¹ is H, (C₁-C₆)alkyl, (C₃-C₆) alkenyl or (C₃-C₆)-alkynyl, where each of the last three radicals mentioned is unsubstituted or substituted by one or more radicals from the group consisting of halogen, phenyl, (C₁-C₄)alkoxy, (C₁-C₄)alkylthio and [(C₁-C₄)alkoxy] -carbonyl, or (C₃-C₆) cycloalkyl, (C₃-C₆) cycloalkyl (C₁-C₃) alkyl, heterocyclyl having 3 to 6 ring atoms or heterocyclyl-(C₁-C₃)alkyl having 3 to 6 ring atoms, where each of the last 4 radicals mentioned is unsubstituted or substituted by one or more radicals from the group consisting of halogen, (C₁-C₄)alkyl and (C₁-C₄)alkoxy,
R² is phenyl, unsubstituted or substituted by one or more radicals from the group consisting of (C₁-C₄)alkyl, (C₁-C₄)haloalkyl, (C₁-C₄)haloalkoxy, (C₁-C₄)alkoxy and halogen, and
R³ is (C₃-C₆)cycloalkyl, unsubstituted or substituted by one or more radicals from the group consisting of halogen, (C₁-C₄)alkoxy, (C₁-C₄)alkylthio, [(C₁-C₄)alkoxy]carbonyl, phenyl and (C₁-C₄)alkyl, or
R² is H or (C₁-C₄)alkyl and
R³ is phenyl, which is substituted by one or more radicals from the group consisting of (C₁-C₄)haloalkyl, or
R² is (C₁-C₄)alkyl and
R³ is CF₃, or
R² and R³, together with the carbon atom, are a ring having 4-8 ring atoms, which is carbocyclic or heterocyclic with one or two heteroatoms from the group consisting of N, O and S and is unsubstituted or substituted by one or more radicals from the group consisting of (C₁-C₄)-alkyl, halogen and oxo, and
R⁴ is H or (C₁-C₆)alkyl, which is unsubstituted or substituted by one or more radicals from the group consisting of halogen, (C₁-C₄)alkoxy, (C₁-C₄)alkylthio, (C₁-C₄)alkylsulfonyl, [(C₁-C₄) alkoxy] carbonyl, CN, phenyl and (C₃-C₆) cycloalkyl, or (C₃-C₆)alkenyl or (C₃-C₆)-alkynyl, where each of the last two radicals mentioned is unsubstituted or substituted by one or more halogen atoms, or a group of the formula 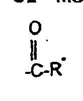 or -SO₂R**
R* is H, (C₁-C₈) alkyl, (C₂-C₆) alkenyl or (C₂-C₆)-alkynyl, where each of the last three radicals mentioned is unsubstituted or substituted by one or more radicals from the group consisting of halogen, (C₁-C₄) alkoxy, (C₁-C₄) alkylthio, phenoxy, [(C₁-C₄) alkoxy] carbonyl, unsubstituted or substituted heterocyclyl and unsubstituted or substituted phenyl, or unsubstituted or substituted (C₃-C₆)cycloalkyl, unsubstituted or substituted phenyl, unsubstituted or substituted heterocyclyl or [(C₁-C₄)alkoxy]carbonyl,
R** is (C₁-C₆)alkyl, (C₃-C₆)alkenyl or (C₃-C₆)alkynyl, where each of the last three radicals mentioned is unsubstituted or substituted by one or more radicals from the group consisting of halogen, (C₁-C₄)alkoxy, (C₁-C₄)alkylthio and phenyl, or phenyl, which is unsubstituted or substituted by one or more radicals from the group consisting of halogen, CN, NO₂, (C₁-C₄)alkyl, (C₁-C₄)haloalkyl and (C₁-C₄)alkoxy,
W is O or S,
X and Y independently of one another are H, halogen, (C₁-C₄)alkyl, (C₁-C₄)alkoxy or (C₁-C₄)alkylthio, where each of the last three radicals mentioned is unsubstituted or substituted by one or more radicals from the group consisting of halogen, (C₁-C₃)alkoxy and (C₁-C₄)alkylthio, or mono- or di[(C₁-C₄)alkyl]-amino, (C₃-C₆)-cycloalkyl, (C₃-C₅)alkenyl, (C₃-C₅)alkenyloxy or (C₃-C₅)alkynyloxy and
Z is CH or N.

3. A compound or salt thereof as claimed in claim 1 or 2, in which
R¹ is (C₁-C₆)alkyl, which is unsubstituted or substituted by one or more radicals from the group consisting of halogen and (C₁-C₄)alkoxy, or (C₃-C₄) alkenyl or (C₃-C₄) alkynyl, or heterocyclyl having 3 to 6 ring atoms and 1 or 2 hetero ring atoms from the group consisting of N and O,
R² is phenyl, which is substituted by one or more radicals from the group consisting of (C₁-C₄)-alkyl, (C₁-C₄)alkoxy and halogen, and
R³ is (C₃-C₆)cycloalkyl, which is substituted by one or more radicals from the group consisting of fluorine, bromine, CN, NO₂, (C₁-C₄)haloalkyl, (C₁-C₄)alkoxy, (C₁-C₄)alkylthio and [(C₁-C₄)-alkoxy]carbonyl, or
R² is H or (C₁-C₄)alkyl and
R³ is phenyl, which is substituted by one or more radicals from the group consisting of (C₁-C₄)haloalkyl, or
R² is (C₁-C₄)alkyl and
R³ is CF₃, or
R² and R³, together with the carbon atom, are a ring having 4-8 ring members, which is carbocyclic or heterocyclic with a heteroatom from the group consisting of N, O and S and is unsubstituted or mono- or polysubstituted by (C₁-C₄)alkyl, halogen or an oxo group, and
R⁴ is H, (C₁-C₄) alkyl, which is unsubstituted or substituted by one or more radicals from the group consisting of halogen, (C₁-C₄)alkoxy, (C₁-C₄)alkylthio, [(C₁-C₄)alkoxy]carbonyl and phenyl, or (C₃-C₄)alkenylor (C₃-C₄)alkynyl,
R⁵ is H, (C₁-C₄) alkyl, (C₁-C₄)haloalkyl, (C₁-C₄)-alkoxy or halogen,
X and Y independently of one another are (C₁-C₄)-alkyl or (C₁-C₄)alkoxy, where each of the last two radicals mentioned are unsubstituted or substituted by one or more halogen atoms, or (C₁-C₄)alkylthio, halogen or mono- or di[(C₁-C₂)-alkyl]amino and
W is O.

4. A compound or salt thereof as claimed in one of claims 1 to 3, in which
R² is H or (C₁-C₄)alkyl and
R³ is phenyl, which is substituted by one or more radicals from the group consisting of (C₁-C₄)haloalkyl, or
R² and R³, together with the carbon atom, are a ring having 5 or 6 ring members, which can optionally contain a heteroatom from the group consisting of N, O and S and is unsubstituted or mono- or polysubstituted by (C₁-C₂)alkyl, and
R⁴ is H,
R⁵ is H, (C₁-C₄)alkyl or halogen,
X is (C₁-C₂)alkyl, (C₁-C₂)alkoxy, (C₁-C₂)alkylthio, (C₁-C₂)haloalkyl or (C₁-C₂)haloalkoxy and
Y is (C₁-C₂)alkyl, (C₁-C₂)alkoxy, halogen, NHCH₃ or N(CH₃)₂.

5. A process for the preparation of a compound of the formula (I) or salt thereof as claimed in claim 1, which comprises
a) reacting a compound of the formula (II) 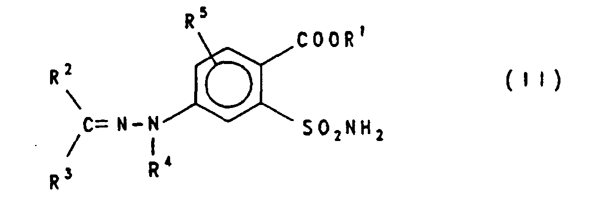 with a heterocyclic carbamate of the formula (III) 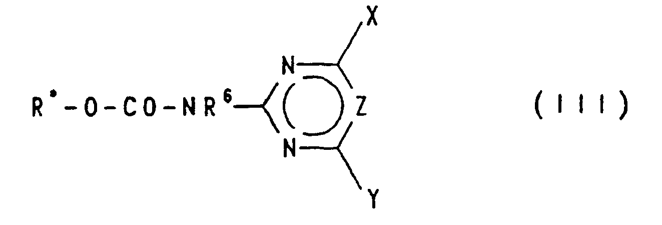 in which R* is optionally substituted phenyl or (C₁-C₄) alkyl, or
b) reacting a sulfonylcarbamate of the formula (IV) 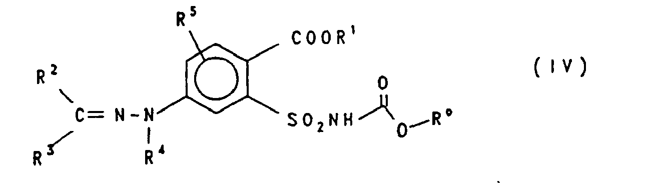 in which R⁰ is unsubstituted or substituted phenyl or (C₁-C₄)alkyl, with an amino-heterocyclic compound of the formula (V) 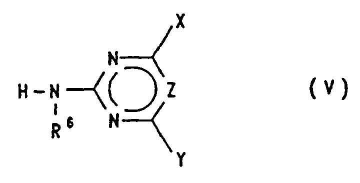 or
c) reacting a sulfonyl isocyanate of the formula (VI) 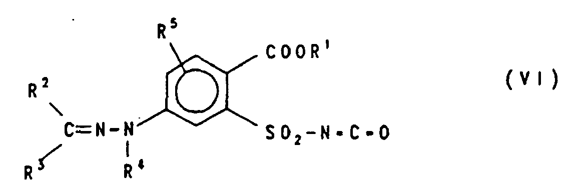 with an amino-heterocyclic compound of the formula (V), or
d) reacting a sulfonamide of the formula (II) with a (thio)isocyanate of the formula (VII) 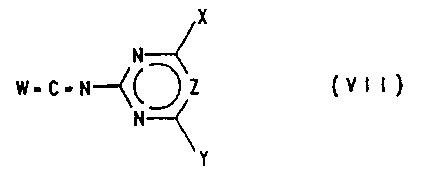 in the presence of a base, or
e) first reacting an amino-heterocyclic compound of the formula (V) with a carbonic acid ester, under base catalysis and reacting the intermediate formed with a sulfonamide of the formula (II) in a one-pot reaction,
in which, in the formulae (II)-(VII), the radicals and groups R¹-R⁶, W, X, Y and Z are defined as in formula (I) and in process variants a) to c) and e) compounds (I) where W = O are initially obtained.

6. A herbicidal or plant growth-regulating composition, which comprises at least one compound of the formula (I) or a salt thereof as claimed in one of claims 1 to 4 and formulating auxiliaries customary in plant protection.

7. A method of controlling harmful plants or of regulating the growth of plants, which comprises applying an active amount of at least one compound of the formula (I) or of a salt thereof as claimed in one of claims 1 to 4 to the harmful plant or plants, plant seeds thereof or the area on which they grow.

8. The use of a compound of the formula (I) or of a salt thereof as claimed in one of claims 1 to 4 as a herbicide or plant growth regulator.

9. A compound of the formula (II) 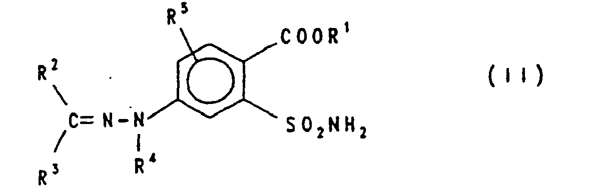 in which R¹ to R⁵ are defined as in formula (I) as claimed in one of claims 1 to 4.

## Revendications

1. Composés de formule (I) ou leurs sels, 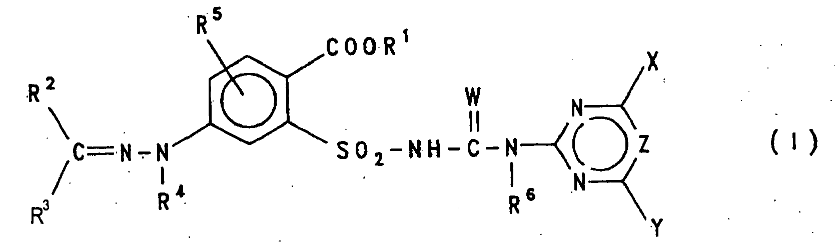 où
R¹ représente un atome d'hydrogène ou un reste hydrocarboné, qui est non substitué ou substitué, ou un reste hétérocyclyle, qui est non substitué ou substitué,
R² représente un groupe phényle non substitué ou substitué, et
R³ représente un groupe cycloalkyle en C₃-C₆ non substitué ou substitué, ou
R² représente un atome d'hydrogène ou un groupe alkyle en C₁-C₄ et
R³ représente un groupe phényle, qui est substitué par un ou plusieurs restes pris dans le groupe constitué par haloalkyle en C₁-C₄, ou
R² représente un groupe alkyle en C₁-C₄ et
R³ représente un groupe CF₃ ou
R², R³, conjointement avec l'atome de carbone du groupe méthylène, représente un reste cycloalkylène ou hétérocyclylène à 4 à 8 chaînons, qui est non substitué ou substitué, ainsi que
R⁴ représente un atome d'hydrogène, un reste hydrocarboné aliphatique non substitué ou substitué présentant 1 à 6 atomes de carbone dans le fragment hydrocarboné et un ou plusieurs substituants, les substituants étant pris dans le groupe constitué par halogène, alkoxy en C₁-C₄, (alkyl en C₁-C₄)thio, (alkyl en C₁-C₄)sulfonyle, (alkyl en C₁-C₄)carbonyle, (alkoxy en C₁-C₄)-carbonyle, CN, phényle substitué et non substitué et cycloalkyle en C₃-C₆, ou alcényle en C₂-C₆ ou alcynyle en C₂-C₆ ou un reste acyle,
R⁵ représente un atome d'hydrogène, un atome d'halogène, un groupe NO₂, CN, alkyle en C₁-C₄, alkoxy en C₁-C₄, (alkyl en C₁-C₄)carbonyle ou (alkoxy en C₁-C₄)carbonyle, chacun des quatre derniers restes cités étant non substitué ou substitué dans le fragment alkyle par un ou plusieurs atomes d'halogène,
R⁶ représente un atome d'hydrogène ou un groupe alkyle en C₁-C₄,
W représente un atome d'oxygène ou un atome de soufre,
X, Y, indépendamment l'un de l'autre, représentent un atome d'hydrogène, un atome d'halogène, un groupe alkyle en C₁-C₄, alkoxy en C₁-C₄, (alkyl en C₁-C₄)thio, chacun des trois derniers restes cités étant non substitué ou substitué par un ou plusieurs restes pris dans le groupe parmi halogène, alkoxy en C₁-C₄ et (alkyl en C₁-C₄) thio, ou mono- ou di-(alkyl en C₁-C₄)-amino, cycloalkyle en C₃-C₆, alcényle en C₂-C₅, alcynyle en C₂-C₅, alcényloxy en C₂-C₅ ou alcynyloxy en C₂-C₅, et
Z représente un groupe CH ou N.

2. Composés et leurs sels selon la revendication 1, **caractérisés en ce que**
R¹ représente un atome d'hydrogène, un groupe alkyle en C₁-C₆, alcényle en C₃-C₆, alcynyle en C₃-C₆, chacun des trois derniers restes cités étant non substitué ou substitué par un ou plusieurs restes pris dans le groupe constitué par halogène, phényle, alkoxy en C₁-C₄, (alkyl en C₁-C₄)-thio et (alkoxy en C₁-C₄)carbonyle, ou cycloalkyle en C₃-C₆, (cycloalkyl en C₃-C₆)-alkyle en C₁-C₃, hétérocyclyle à 3 à 6 chaînons ou hétérocyclyl (alkyle en C₁-C₃) présentant 3 à 6 chaînons, chacun des 4 derniers restes cités étant non substitué ou substitué par un ou plusieurs restes pris dans le groupe constitué par halogène, alkyle en C₁-C₄ et alkoxy en C₁-C₄,
R² représente un groupe phényle non substitué ou substitué par un ou plusieurs restes pris dans le groupe constitué par alkyle en C₁-C₄, haloalkyle en C₁-C₄, haloalkoxy en C₁-C₄, alkoxy en C₁-C₄ et halogène, et
R³ représente un groupe cycloalkyle en C₃-C₆ non substitué ou substitué par un ou plusieurs restes pris dans le groupe constitué par halogène, alkoxy en C₁-C₄, (alkyl en C₁-C₄)thio, (alkoxy en C₁-C₄)carbonyle, phényle et alkyle en C₁-C₄, ou
R² représente un atome d'hydrogène ou un groupe alkyle C₁-C₄, et
R³ représente un groupe phényle, qui est substitué par un ou plusieurs restes pris dans le groupe constitué par haloalkyle en C₁-C₄, ou
R² représente un groupe alkyle en C₁-C₄ et
R³ représente un groupe CF₃ ou
R², R³, conjointement avec l'atome de carbone, forment un cycle à 4 à 8 chaînons, qui est carbocyclique ou hétérocyclique présentant un ou deux hétéroatomes pris dans le groupe des atomes de N, O et S et qui est non substitué ou substitué par un ou plusieurs restes pris dans le groupe constitué par alkyle en C₁-C₄, halogène et oxo, ainsi que
R⁴ représente un atome d'hydrogène ou un groupe alkyle en C₁-C₆, qui est non substitué ou substitué par un ou plusieurs restes pris dans le groupe constitué par halogène, alkoxy en C₁-C₄, (alkyl en C₁-C₄)thio, (alkyl en C₁-C₄)sulfonyle, (alkoxy en C₁-C₄)carbonyle, CN, phényle et cyclkoalkyle en C₃-C₆, ou un groupe alcényle en C₃-C₆ ou alcynyle en C₃-C₆, chacun des deux derniers restes cités étant non substitué ou substitué par un ou plusieurs atomes d'halogène, ou un groupe de formule 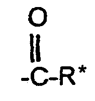 ou -SO₂R**
R* représente un atome d'hydrogène, un groupe alkyle en C₁-C₈, alcényle en C₂-C₆, alcynyle en C₂-C₆, chacun des trois derniers restes cités étant non substitué ou substitué par un ou plusieurs restes pris dans le groupe constitué par halogène, alkoxy en C₁-C₄, (alkyl en C₁-C₄)thio, phénoxy, (alkoxy en C₁-C₄)carbonyle, hétérocyclyle non substitué ou substitué et phényle non substitué ou substitué, ou représente cycloalkyle en C₃-C₆ non substitué ou substitué, ou phényle non substitué ou substitué, hétérocyclyle non substitué ou substitué ou (alkoxy en C₁-C₄)carbonyle,
R** représente un groupe alkyle en C₁-C₆, alcényle en C₃-C₆, alcynyle en C₃-C₆, chacun des trois derniers restes cités étant non substitué ou substitué par un ou plusieurs restes pris dans le groupe constitué par halogène, alkoxy en C₁-C₄, (alkyl en C₁-C₄)thio et phényle, ou phényle qui est non substitué ou substitué par un ou plusieurs restes pris dans le groupe parmi halogène, CN, NO₂, alkyle en C₁-C₄, haloalkyle en C₁-C₄ et alkoxy en C₁-C₄,
W représente un atome d'oxygène ou de soufre,
X et Y, indépendamment l'un de l'autre, représentent un atome d'hydrogène, un atome d'halogène, un groupe alkyle en C₁-C₄, alkoxy en C₁-C₄, (alkyl en C₁-C₄)thio, chacun des trois derniers restes cités étant non substitué ou substitué par un ou plusieurs restes pris dans le groupe constitué par halogène, alkoxy en C₁-C₃ et (alkyl en C₁-C₄)-thio, mono- ou di-(alkyl en C₁-C₄)-amino, cycloalkyle en C₃-C₆, alcényle en C₃-C₅, alcényloxy en C₃-C₅ ou alcynyloxy en C₃-C₅, et
Z représente un groupe CH ou N.

3. Composés et leurs sels selon la revendication 1 ou 2, **caractérisés en ce que**
R¹ représente un groupe alkyle en C₁-C₆, qui est non substitué ou substitué par un ou plusieurs restes pris dans le groupe constitué par halogène et alkoxy en C₁-C₄, ou alcényle en C₃-C₄ ou alcynyle en C₃-C₄, ou hétérocyclyle à 3 à 6 chaînons et 1 ou 2 chaînons hétéroatomes pris dans le groupe constitué par les atomes de N et O,
R² représente un groupe phényle, qui est substitué par un ou plusieurs restes pris dans le groupe constitué par alkyle en C₁-C₄, alkoxy en C₁-C₄ et halogène, et
R³ cycloalkyle en C₃-C₆, qui est substitué par un
ou plusieurs restes pris dans le groupe constitué par fluor, brome, CN, NO₂, haloalkyle en C₁-C₄, alkoxy en C₁-C₄, (alkyl en C₁-C₄)thio et (alkoxy en C₁-C₄)carbonyle, ou
R² représente un atome d'hydrogène ou un groupe alkyle en C₁-C₄, et
R³ représente un groupe phényle, qui est substitué par un ou plusieurs restes pris dans le groupe constitué par haloalkyle en C₁-C₄, ou
R² représente alkyle en C₁-C₄ et
R³ représente un groupe CF₃ ou
R², R³, conjointement avec l'atome de carbone, forment un cycle à 4 à 8 chaînons, qui est carbocyclique ou hétérocyclique avec un hétéroatome pris dans le groupe des atomes de N, O et S, et est non substitué ou substitué une ou plusieurs fois par un groupe alkyle en C₁-C₄, halogène ou oxo, et
R⁴ représente un atome d'hydrogène, un groupe alkyle en C₁-C₄, qui est non substitué ou substitué par un ou plusieurs restes pris dans le groupe constitué par halogène, alkoxy en C₁-C₄, (alkyl en C₁-C₄)thio et (alkoxy en C₁-C₄)-carbonyle et phényle, ou alcényle en C₃-C₄ ou alcynyle en C₃-C₄,
R⁵ représente un atome d'hydrogène, un groupe alkyle en C₁-C₄, haloalkyle en C₁-C₄, alkoxy en C₁-C₄ ou halogène,
X et Y, indépendamment l'un de l'autre, représentent un groupe alkyle en C₁-C₄, alkoxy en C₁-C₄, chacun des deux derniers restes cités étant non substitué ou substitué par un ou plusieurs atomes d'halogène, ou un groupe (alkyl en C₁-C₄)-thio, halogène ou mono- ou di-(alkyl en C₁-C₂)-amino, et
W représente un atome d'oxygène.

4. Composés et leurs sels selon l'une des revendications 1 à 3, **caractérisés en ce que**
R² représente un atome d'hydrogène ou un groupe alkyle en C₁-C₄ et
R³ représente un groupe phényle, qui est substitué par un ou plusieurs restes pris dans le groupe constitué par haloalkyle en C₁-C₄, ou
R² et R³, conjointement avec l'atome de carbone, forment un cycle à 5 ou 6 chaînons, qui éventuellement peut présenter un hétéroatome pris dans le groupe des atomes de N, O et S et qui est non substitué ou substitué une ou plusieurs fois par un groupe alkyle en C₁-C₂, ainsi que
R⁴ représente un atome d'hydrogène,
R⁵ représente un atome d'hydrogène, un groupe alkyle en C₁-C₄ ou halogène,
X représente un groupe alkyle en C₁-C₂, alkoxy en C₁-C₂, (alkyl en C₁-C₂)thio, haloalkyle en C₁-C₂, ou haloalkoxy en C₁-C₂, et
Y représente un groupe alkyle en C₁-C₂, alkoxy en C₁-C₂, halogène, NHCH₃ ou N(CH₃)₂.

5. Procédé pour la préparation de composés de formule (I) ou leurs sels selon la revendication 1, **caractérisé en ce que**
a) on fait réagir un composé de formule (II) 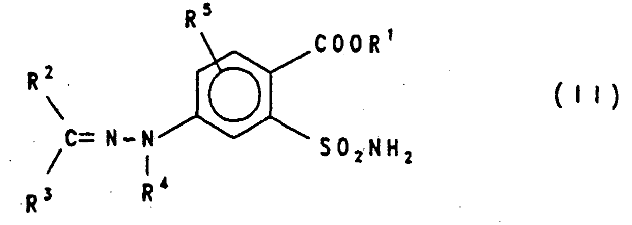 sur un carbamate hétérocyclique de formule (III) 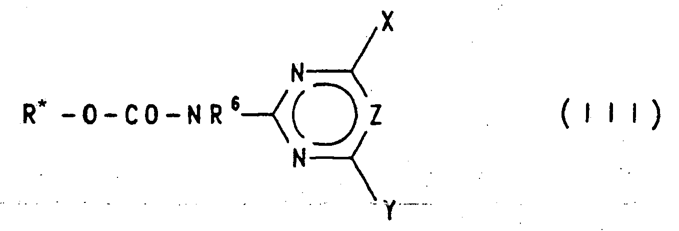 où R* représente un groupe alkyle en C₁-C₄ ou phényle éventuellement substitué, ou
b) on fait réagir un sulfonylcarbamate de formule (IV) 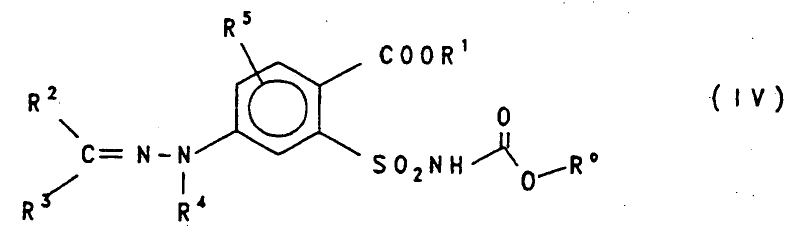 où R^{o} représente un groupe alkyle en C₁-C₄ ou phényle non substitué ou substitué, sur un aminohétérocycle de formule (V) 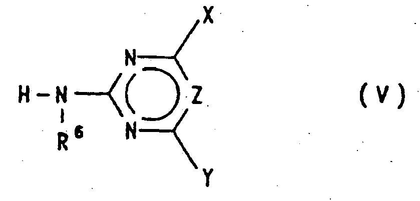 ou
c) on fait réagir un sulfonylisocyanate de formule (VI) 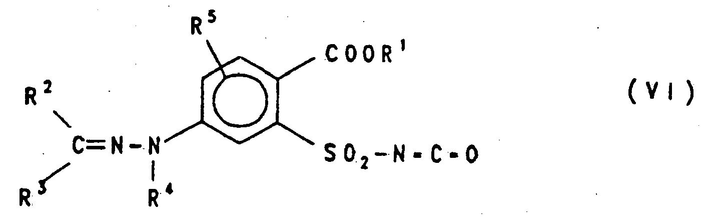 sur un aminohétérocycle de formule (V) ou
d) on fait réagir un sulfonamide de formule (II) sur un (thio)-isocyanate de formule (VII) 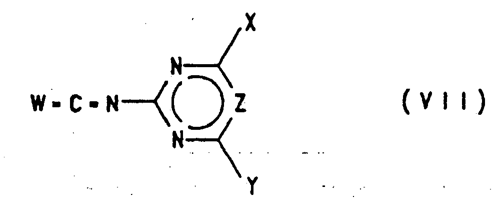 en présence d'une base, ou
e) on fait réagir un aminohétérocycle de formule (V) d'abord catalysé par une base sur un ester de l'acide carbonique, et l'intermédiaire formé dans une réaction à un pot sur un sulfonamide de formule (III),
dans les formules (II)-(VII) les restes ou les groupes R¹-R⁶, W, X, Y et Z sont définis comme à la formule (I) et on obtient dans les variantes de procédé a) à c) d'abord les composés (I) avec W = O.

6. Agent herbicide ou régulateur de croissance **caractérisé en ce qu'**il renferme au moins un composé de formule (I) ou son sel selon l'une des revendications 1 à 4 et des formulants usuels dans le domaine de la phytoprotection.

7. Procédé pour lutter contre les plantes adventices ou pour la régulation de croissance de plantes **caractérisé en ce qu'**on applique une quantité efficace d'au moins un composé de formule (I) ou son sel selon l'une des revendications 1 à 4 aux plantes adventices ou plantes, à leur semence ou à la surface cultivable.

8. Utilisation des composés de formule (I) ou leurs sels selon l'une des revendications 1 à 4 en tant qu'herbicides ou régulateurs de croissance de plantes.

9. Composés de formule (II) 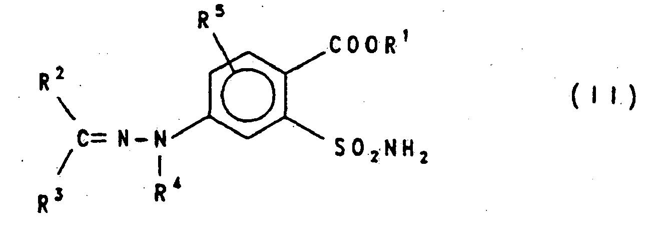 où R¹ à R⁵ sont définis comme à la formule (I) selon l'une des revendications 1 à 4.
